# EUROPEAN PATENT APPLICATION

(11) **EP 4 368 248 A1**
(43) Date of publication of application: **15.05.2024**
(21) Application number: 23208551.4
(22) Date of filing: 08.11.2023
(51) Int. Cl.: A61N 5/10

(54) **AN IMPROVED BORE DESIGN FOR A RADIOTHERAPY DEVICE**

(30) Priority: 08.11.2022 GB 202216613
(71) Applicant: Elekta Limited, Crawley, Sussex RH10 9RR (GB)
(72) Inventor: CARLANDER, Erik, Crawley, RH10 9RR (GB)
(74) Representative: Collins, Emily Jane

(57) **Abstract**

Disclosed herein is a radiotherapy device comprising a fixed support structure; and an inner cover defining a bore, wherein the inner cover is movable with respect to the first support structure from a first position.

## Description

This disclosure relates generally to radiotherapy, and in particular to an improved bore design for a radiotherapy device.

### Background

Radiotherapy uses ionising radiation to treat a human or animal body. In particular, radiotherapy is commonly used to treat tumours within a body. In such treatments, cells forming part of the tumour are irradiated by ionising radiation in order to destroy or damage them. However, in order to apply a prescribed dose of ionising radiation to a target location or a target region, such as a tumour, the ionising radiation will typically also pass through healthy tissue of the body.

Modern radiotherapy treatment uses techniques to reduce the radiation dose to healthy tissue and thereby provide a safe treatment. For example, one approach to minimising a radiation dose received by healthy tissue surrounding a target region is to direct the radiation towards the target region from a plurality of different angles, for example by rotating a source of radiation around the patient by use of a rotating gantry. In this case, the angles at which radiation is applied are selected such that each beam of radiation passes through the target region. In this way, a cumulative radiation dose may be built up at the target region over the course of a treatment delivery in which the radiation source rotates through a certain angle. However, since the radiation is applied from a plurality of different angles, the same, high, cumulative radiation dose is not built up in the healthy tissue because the specific healthy tissue the radiation passes through varies with angle. Therefore, a unit volume of the healthy tissue receives a reduced radiation dose relative to a unit volume of the target region.

In radiotherapy it is important to take steps to reduce patient discomfort to improve the patient's experience. It is also important to lower patient anxiety, since stress is damaging to patient health and may negatively affect the radiotherapy treatment. Seeing the machinery of the radiotherapy device during treatment may be intimidating to the patient and cause the patient stress. Therefore, most radiotherapy devices comprise an inner cover defining a bore inside the gantry. When being treated, the patient is positioned inside the inner cover and views the inner cover instead of any machinery.

### Summary

An invention is set out in the claims.

According to an aspect there is provided a radiotherapy device comprising: an inner cover defining a bore, wherein the inner cover is movable from a first position.

According to an aspect there is provided a radiotherapy device comprising: a fixed support structure; and an inner cover defining a bore, wherein the inner cover is movable with respect to the first support structure from a first position.

Optionally, the radiotherapy device further comprises a rotatable ring-shaped gantry.

Optionally, the inner cover is suspended relative to the support structure by a suspension system.

Optionally, the suspension system comprises one or more of: one or more springs, and one or more deformable leg supports.

Optionally, the inner cover is movable from the first position in two dimensions, in the plane of the circular cross section of the inner cover.

Optionally, the radiotherapy device further comprises at least one module.

Optionally, the radiotherapy device further comprises at least one module comprising one or more biasing mechanisms configured to return the inner cover to the first position.

Optionally, the one or more biasing mechanisms are return springs.

Optionally, the at least one module further comprises one or more end stops, wherein the one or more end stops are configured to prevent the inner cover from being displaced beyond a threshold distance.

Optionally, the at least one module further comprises a sensor configured to provide displacement values indicative of the displacement of the inner cover from the first position.

Optionally, the radiotherapy device comprises at least one sensor configured to provide displacement values indicative of the displacement of the inner cover from the first position.

Optionally, the sensors are configured to measure displacement from the first position of the inner cover in one dimension.

Optionally, the sensor is configured to measure displacement from the first position of the inner cover in either a horizontal or vertical direction with respect to the radiotherapy device support structure.

Optionally, the sensor is one of: a linear potentiometer, an inductive sensor, a capacitive sensor or a switch sensor.

Optionally, the sensor is configured to measure displacement from the first position of the inner cover in two dimensions.

Optionally, the sensor is a photoelectric sensor, a time of flight camera, a contact sensor or a theremin sensor.

Optionally, the sensor is a contact-less sensor.

Optionally, the at least one module comprises at least part of the sensor.

Optionally, the radiotherapy device comprises a plurality of modules.

Optionally, the radiotherapy device comprises seven modules.

Optionally, the radiotherapy device further comprises a pivot point for pivotally restricting movement of the inner cover relation to the support structure.

Optionally, the radiotherapy device further comprises a pivot point wherein the pivot point comprises: a divot positioned on the fixed support structure; and a protrusion from the inner cover, wherein the protrusion can fit in the divot.

Optionally, the radiotherapy device further comprises four modules at a front end of the inner cover, three modules at a rear end of the inner cover and one pivot point at the rear end of the inner cover.

Optionally, the radiotherapy device further comprises a first plurality of modules aligned in a first direction and a second plurality aligned in a second direction perpendicular to the first direction.

Optionally, the radiotherapy device further comprises a fixed support structure, wherein a first part of the module is fixedly attached to the inner cover and a second part of the module is fixedly attached to the support structure, wherein the first and second parts of the module are configured such that displacement of the inner cover with respect to the support structure causes displacement of the first part with respect to the second part.

Optionally, the sensor measures displacement of the first part relative to the second part such that the sensor measures displacement of the inner cover from the first position of the inner cover.

Optionally, the radiotherapy device further comprises a sensor configured to provide displacement values indicative of the displacement of the inner cover from the first position .

Optionally, the module further comprises one or more biasing mechanisms configured to return the inner cover to the first position.

Optionally, the module further comprises one or more end stops, the one or more end stops configured to prevent the inner cover from being displaced beyond a threshold distance.

According to an aspect there is provided a method for a radiotherapy device, the radiotherapy device according to any preceding claim, the method comprising: detecting displacement of the inner cover from the first position by one or more sensors, wherein the sensor provides displacement values indicative of displacement of the inner cover; processing the one or more displacement values, wherein the processing comprises comparing the displacement values to one or more thresholds; based on the processing of the displacement values, actioning one or more next steps.

Optionally, the method further comprises wherein if the displacement value is more than a threshold value and the patient table is in motion, the one or more next steps comprises stopping the motion of the patient table.

Optionally, the method further comprises wherein if the displacement value is more than a threshold value and the gantry is rotating, the one or more next step comprises stopping the gantry rotation.

Optionally, the method further comprises wherein if the displacement value is less than a threshold value, maintain the device as normal and send a warning message to the user.

### Figures

Specific embodiments are now described, by way of example only, with reference to the drawings, in which:
Figure 1 depicts a radiotherapy device.
Figures 2a to 2b depict part of a radiotherapy device according to the present disclosure.
Figure 3 depicts an example pivot point according to the present disclosure.
Figure 4 depicts example deformable leg supports according to the present disclosure.
Figure 5 depicts an example radiotherapy device comprising a spring and a module according to the present disclosure.
Figure 6 depicts an example module 600 according to the present disclosure.
Figures 7a to 7b depict example end positions of two end stops according to the present disclosure.
Figures 8a and 8b depict the pivot point of Figure 3 further comprising a sensor stops according to the present disclosure.
Figure 9a depicts an example radiotherapy device with an optical sensor according to the present disclosure.
Figure 9b and 9c depict an example optical sensor according to the present disclosure.
Figure 10a depicts an example radiotherapy device with an inductive or capacitive sensor according to the present disclosure.
Figure 10b depicts an example radiotherapy device with a contact sensor according to the present disclosure.
Figure 11 depicts a method for a radiotherapy device according to the present disclosure.

### Detailed Description

In presently disclosed methods, an improved inner cover design for a radiotherapy device is provided. If a force to the inner cover occurs, damage to a person or piece of equipment can be avoided. In the following, a device, method and computer-readable medium for an improved inner cover design are provided. The device may be configured to perform any of the method steps presently disclosed and may comprise computer executable instructions which, when executed by a processor, cause a processor to perform any of the method steps presently disclosed. Any of the steps that the device is configured to perform may be considered as method steps of the present disclosure and may be embodied in computer executable instructions for execution by a processor.

In the following, application of radiotherapy to a patient will be referred to in most detail in order to provide clarity of explanation. Such use of the term patient should not be interpreted to limit application of the present disclosure. The present disclosure provides means that can be used to apply radiotherapy to any subject. The terms patient and subject may be used interchangeably herein.

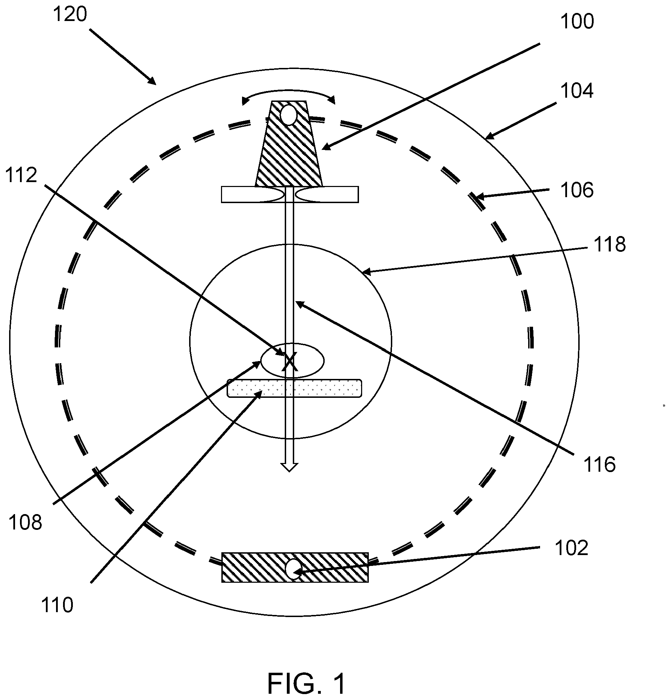

Figure 1 depicts a radiotherapy device. The arrangement described should be considered as providing one or more examples of a radiotherapy device 120 and it will be understood that other arrangements are possible and can be used to perform the methods described herein. The Figure shows a cross-section through a radiotherapy device 120 comprising a radiation source 100 and a detector 102 attached to a rotatable ring-shaped gantry 104. For any reference made herein to a gantry, it can be assumed that the gantry may be rotatable ring shaped gantry. The radiation source 100 and the detector 102 may be fixed to the gantry 104 and may rotate with the gantry. The gantry 104 may comprise a circular support track 106.

Figure 1 also depicts a patient 108 on a table 110. The table 110 may be moved longitudinally relative to the gantry 104 (i.e. away from the plane of the gantry 104), for example to aid positioning of the patient 108. In some examples, the table 110 may be moved along other translational axes (e.g. in the plane of the gantry) and/or rotational axes. The patient table may also be referred to as a moveable or adjustable couch or support surface.

As radiation is applied to the patient 108, for example according to a treatment plan, the radiation source 100 and the detector 102 may rotate together with the gantry 104 and/or around the circular support track 106 such that they are always arranged 180° from one another. The radiation source 100 may direct radiation towards the patient 108 from various angles around the patient 108 in order to spread out the radiation dose received by healthy tissue to a larger region of healthy tissue while building up a prescribed dose of radiation at a target region. As shown in Figure 1, radiation may be emitted in a plane which is perpendicular to the axis of rotation of the radiation source 100. Thus, radiation may be applied to a radiation isocentre 112 at the centre of the gantry 104 regardless of the angle to which the radiation source 100 is rotated around the gantry 104.

In use, the radiotherapy device also comprises an inner cover 118. The inner cover 118 of the rotatable ring-shaped gantry 104 defines a inner cover 118. The inner cover 118 is so-called as it covers an inner surface of the radiotherapy device. The inner cover 118 may be a cylindrical cover. The inner cover 118 may be referred to as a bore tube. The terms inner cover and bore may be used interchangeably herein. The purpose of the inner cover 118 is to cover the components of the radiotherapy device and to shield the patient from the components of the rotating gantry.

The table 110 can be used to move a patient, or other subject, into the inner cover 118 when radiotherapy is to commence. The patient table 110 is configured to move between a first position substantially outside the inner cover 118, and a second position substantially inside the inner cover. In the first position, a patient or subject can mount the patient table. The table 110, and patient, can then be moved inside the inner cover 118, to the second position, in order for the patient to be treated using the radiotherapy device.

In some examples, the radiotherapy device 120 may comprise one or more additional panels and/or sources and/or detectors. The radiotherapy device 120 may comprise one or more imaging devices, which may comprise a source and a detector. This source and detector may be fixed to the gantry 104 and/or may rotate around the circular support track 106 such that they are always 180° from each other. This source and detector may be disposed in the plane of the gantry 104 or may be projected a longitudinal distance from the gantry 104, for example on additional support arms. In some examples, the radiotherapy device 120 may be an MR linac. As used herein, any of the above-described features may be referred to as components of the radiotherapy device. The radiotherapy device 120 may comprise a magnetic resonance (MR) imaging device, which may not comprise a detector separate from a source of the MR imaging device.

A treatment delivery may comprise rotation of the radiation source 100 and application of radiation by the radiation source 100, for example according to a treatment plan. In a treatment delivery, the rotation of the radiation source 100 may be through a predetermined angle. The radiation source 100 may rotate in a continuous or substantially continuous treatment arc, and/or may rotate to and pause at a plurality of discrete angles. The treatment plan comprises a prescribed dose (e.g. a clinically-prescribed dose) for the target region.

As described above, one or more components of the radiotherapy device 120 may move around a patient 108 on the gantry 104 and/or on the circular support track 106. Alternatively, or in addition, the table 110 may move relative to the gantry 104. These movements may occur prior to or during a radiotherapy treatment in order to direct a radiation beam towards the patient 108 from one or more particular angles. Alternatively, or in addition, the patient 108 may move relative to the table 110, and therefore (in general) relative to the other components of the radiotherapy device.

A treatment of radiotherapy may involve various movements in and around the inner cover. Example movements include: movement of the rotating gantry, movement of patient supports or patient tables, movements of the patient and/or movements of the operator of the radiotherapy device. Since radiotherapy treatment may have a duration of the order of seconds or minutes, the patient may move relative to the patient support during the radiotherapy treatment. There are various reasons why a patient may move before, during or after a radiotherapy treatment. For example, the patient may shift position or sit up. Movement of an operator in and around the inner cover may involve the operator reaching towards the patient to re-position the patient or to adjust the patient support.

Due to geometrical and dosimetric considerations, it is often desirable for components of the radiotherapy device to be in close proximity to the patient. Due to this, and to the various movements described above, it is possible that a patient, operator or piece of equipment may come into contact with the inner cover of the gantry. It is also possible that items, such as clothing or accessories, or a patient or operator may become trapped between components of the radiotherapy device and the inner cover. A rigid inner cover may cause the object or person to be pinched or may cause damage to components of the radiotherapy device. This may be cause damage to the patient, operator and/or the radiotherapy device. Such contact may also cause a delay in the application of radiotherapy and/or inaccurate application of radiotherapy due to the associated disruption to the radiotherapy treatment.

It will be appreciated that there is a need for an improved inner cover design for a radiotherapy device. The present disclosure provides means to avoid damage to a person or piece of equipment.

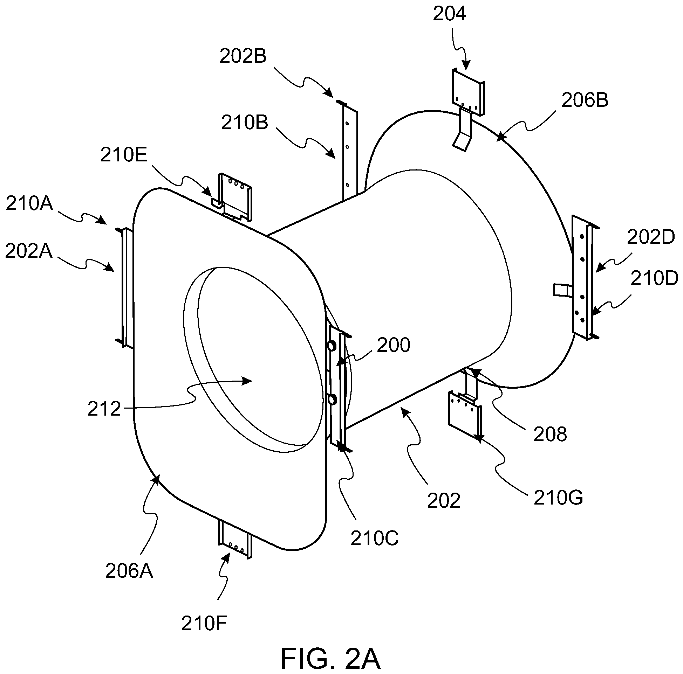

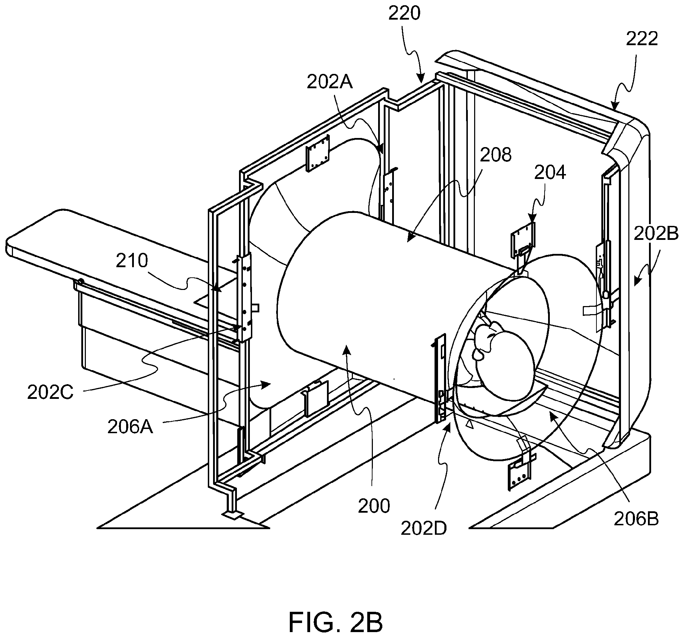

Figures 2a and 2b depict part of an example radiotherapy device according to the present disclosure shown from two different angles.

The radiotherapy device includes a rotatable ring gantry (not shown). The gantry rotates relative to a fixed, stationary support structure. The support structure may be a fixed, stationary structure from which other components of the radiotherapy device may be housed. The support structure may comprise rigid support bars from which other components can attach to, hang from or otherwise be housed inside. The support structure may comprise solid metal support bars, for example steel rods. The support structure 220 is depicted in Figure 2b. The support structure 220 may also include the external fa 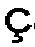 ade of the radiotherapy device.

Figure 2a depicts an inner cover 200 defining a bore 212, wherein the inner cover may be movable from a first position. The defined bore lies through the central hole of the ring-shaped gantry.

In Figure 2b part of the support structure 220 of the radiotherapy device and the table 210 are shown. Figure 2b also depicts some other components of the radiotherapy device, such as a side cover 222. Figure 2b also depicts many of the same components as in Figure 2a, such as the inner cover 200. Reference to these components will now be made with respect to both Figures 2a and 2b.

The inner cover 200 has a 'resting' position which is referred to as a first position. In the first position the cover is neutral. If no external forces are applied to the system, the cover will sit in the first position.

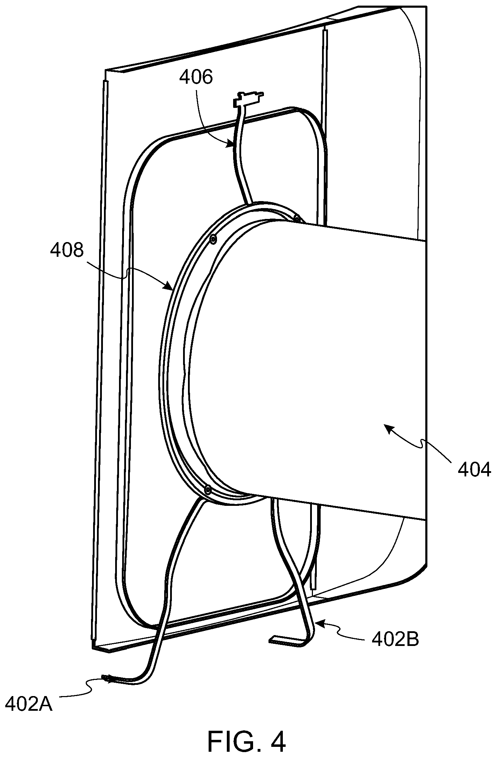

The inner cover is movable from the first position by the application of external forces. That is, the inner cover is not fixedly or rigidly attached to the support structure, rather it is flexibly or moveably attached. For the inner cover 200 to be movable from the first position, the inner cover 200 may be attached to the support structure via a suspension system. For example, the suspension system may comprise one or more springs. As would be appreciated by the skilled person, various suspension systems may be utilized to allow the inner to be moveable from the first position. One example is depicted in Figure 4. Another example is depicted in Figures 2a and 2b.

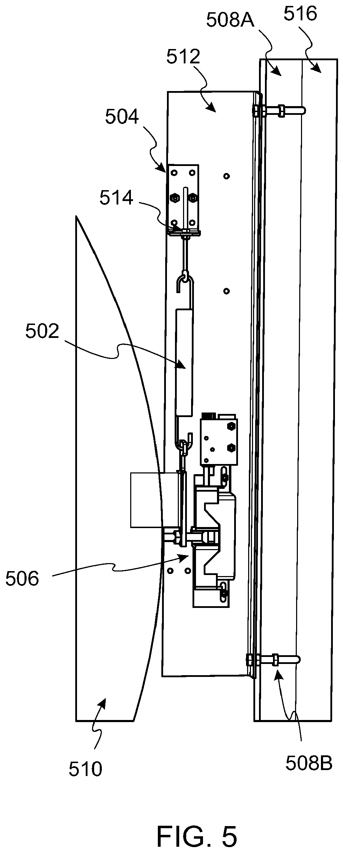

In the examples of Figures 2a and 2b, there are four springs 202A, 202B, 202C, 202D housed in brackets which act as the suspension system. The springs may suspend the inner cover with respect to the radiotherapy device.. The springs 202A, 202B, 202C, 202D may support the weight of the inner cover. Figure 4 illustrates an alternative suspension system. The springs suspension system used in Figures 2a and 2b will be described in more detail in relation to Figure 5.

The inner cover 200 may also comprise two end funnels 206A, 206B. A first end funnel 206A is at the front end of the inner cover and a second end funnel 206B is at the rear end of the inner cover. The front end may be the patient-receiving end. Alternatively, in another embodiment the rear end may be the patient-receiving end. The first and second end funnels 206A, 206B may be considered part of the inner cover 200, such that the first and second end funnels are movable from a first position (as part of the inner cover). The inner cover 200 may be considered as comprising: a cylindrical section 208, a first end funnel 206A and a second end funnel 206B. Therefore the inner cover 200 may also be considered as comprising: a cylindrical section 208, a first end funnel 206A and a second end funnel 206B. In other examples, the inner cover 200 may only comprise a cylindrical section 208. The inner cover may comprise end funnels at each end, the end funnels being attached to a facade of the radiotherapy device. For radiotherapy devices with one or two end funnels, any reference made herein to movement of the inner cover or inner cover may therefore be assumed to be related to the movement of the first and second end funnels.

In the embodiment in Figure 2a and 2b the inner cover is an open-ended tube, with both ends being open. The inner cover is attached to the radiotherapy device housing around the edge of the tube ends, for example at the edges of the end funnel. In this way, the bore defines a through-hole in the radiotherapy device through with a patient can be translated. The bore is open ended at both ends. Other alternative configurations may be envisaged.

The first position of the inner cover 200 is the desired position of the inner cover whilst the patient is treated using the radiotherapy device. If the inner cover is suspended via a suspension system, the first position of the inner cover 200 represents the equilibrium position of the inner cover. The suspension system may be configured such that the inner cover is at rest in the first position. That is, without any force applied to the inner cover, the inner cover should remain in the first position. The inner cover may be moveable from the first position by use of a suspension system. Movement of the inner cover from a first position may involve movements of the inner cover in any direction. The directions of movements of the inner cover may depend on the design of the suspension system. Some designs may only allow movement in certain directions.

In another example, the inner cover is movable form the first position by virtue of deformable or flexible leg supports. That is, the suspension system need not be springs affixed between the support structure and the inner cover. This is illustrated in Figure 4.

Figures 2a and 2b also depict seven modules 210 according to the present disclosure. The seven modules are located such that there are four at the front end of the inner cover and three modules at the rear end of the inner cover. Four modules are aligned in a vertical direction 210A, 210B, 210C, 210D and three modules are aligned in a horizontal direction 210E, 210F, 210G. The modules will be later discussed in detail with reference to Figures 5, 6, 7a, 7b and 9.

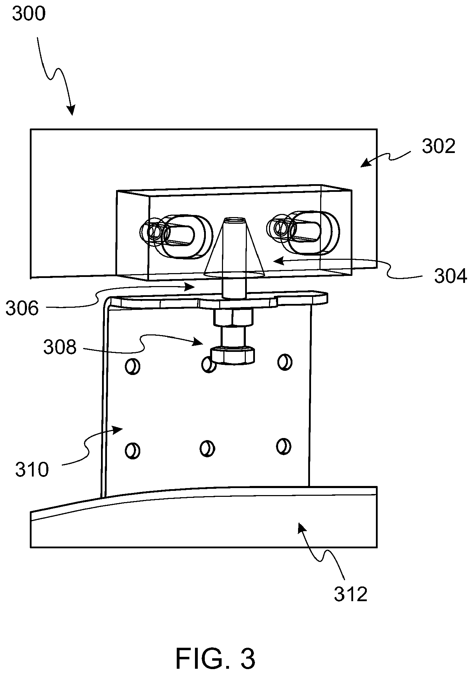

Figures 2a and 2b also depict a pivot point 204 which may act as an anchor point, anchoring the inner cover 200 to the support structure (not shown in Figure 2a). The pivot point comprises a divot positioned on the support structure, and a protrusion from the inner cover. The inner cover 200 is not attached to the support structure at the pivot point, but rather part of the inner cover, such as the protrusion, is positioned in the divot. The pivot point permits movement of the inner cover relative to the support structure in some directions, but limits or restricts movement of some parts of the inner cover relative to the support structure. This is explained below in relation to Figure 3.

Figure 3 depicts a specific example of a pivot point 300. The pivot point comprises a divot positioned on the support structure, and a protrusion from the inner cover. The protrusion can fit in the divot. When the inner cover is in the first position, the suspension system biases the protrusion into the divot. The divot is cone-shaped, such that when the protrusion is biased into the divot, it is guided towards the top point of the divot. In this position, referred to as the engaged position, the divot 204 may restrict the movement of the inner cover 200 in the sense that the inner cover cannot move in the horizontal plane relative to the support structure at the end at which the divot/protrusion are located. This can help protect against any movement which might otherwise happen given than the inner cover is not fixedly attachment to the support structure. Nonetheless, vertical movement of the inner cover 200 downwards from the engaged position is permitted. Vertical movements may be vertical with respect to the stationary support structure 220 of the radiotherapy device.

In the example depicted in Figure 2a, the pivot point 204 is positioned at the rear end of the inner cover, at the top of the second end funnel 206B. It will be appreciated that the divot could be positioned at the front end on the inner cover.

When in the engaged position the inner cover may pivot around the divot 204. That is, the inner cover may be permitted to move in a horizontal plane at the end opposing that to the pivot point. Horizontal movement of the inner cover at the end having the pivot point is restricted. In the example in Figure 2, the pivot horizontal movement of the inner cover at the rear end is restricted. The protrusion is positioned in the divot, and the suspension system biases the protrusion towards the point of the divot, which restricts horizontal movement of the protrusion, and therefore of the rear end of the inner cover. However, movement of the front end of the cover is not restricted. The front end of the cover can move in the horizontal (and vertical) direction relative to the support structure. When the front end of the inner cover moves in a horizontal direction (for example through contact with a patient), horizontal movement of the rear end of the inner cover is not permitted. This leads to the inner cover 'pivoting' around the pivot point and with relation to the support structure.

A specific example of a pivot point is illustrated in Figure 3. Figure 3 depicts a first bracket 302 which is fixedly attached to the support structure. Fixedly attached to the first bracket 302 is a block having a conical divot 304. That is, a solid block piece of material may be partially hollowed out, the hollowing out in the shape of a cone. As will be appreciated, the block could be made from most metals and may be milled or casted. The block piece may alternatively be made from plastic, for example milled from a plastic block or made by moulding. In some examples, the block could also be made from composite material, fiberglass and epoxy. The hollowing out in the shape of a cone is such that the conical divot 304 can receive a protrusion from the inner cover, such as screw 306. The screw 306 fits within the conical divot 304, the screw is not fixedly attached to the conical divot 304.

The inner cover comprises a protrusion, which may take the form of a screw 306 fixedly attached to a second bracket 310. The screw 306 may be secured by a nut 308. The second bracket 310 is fixedly attached to the inner cover 312. The first bracket 302 and conical divot 304 remain in a stationary position since they are fixedly attached to the stationary support structure of the radiotherapy device. The screw 306, nut 308 and second bracket 302 are fixedly attached to inner cover 312 and therefore move with the inner cover, moveable from a first position. The screw 306, nut 308, second bracket 302 may be considered as part of the inner cover 312.

The screw 306 may move in any direction allowed by the conical divot 304. The conical divot 304 may allow the screw 306 to pivot. The centre of rotation of the pivot would be the top of the cone. The screw may pivot in any direction allowed by the conical divot 304. The screw 306 may also move vertically, if the screw moves vertically the screw may either move further inside the conical divot 304 or may move towards the outside the cone. The conical divot 304 may be a hollow conical frustum shape. To form a conical frustum the top of the cone is sliced off, the top of the cone is sliced off at a similar radius to the radius of the screw 306. This is to allow the screw to move vertically.

There may also be a hollow shaft extending from the top of the cone to allow for vertical movement of the screw 306, the hollow shaft of a similar diameter to the radius of the screw.

The screw 306 may move vertically such that the screw is not within the conical divot 304, this movement would be used when the inner cover 312 is removed from the radiotherapy device for servicing. After servicing is complete, the screw 306 may be aligned with the conical divot 304 and be received by the conical divot 304. In normal operation of the radiotherapy device, the vertical movements of the inner cover 312 may be restricted such that the screw 306 cannot move outside the conical divot 304.

The pivot point 300 may be considered as comprising a divot on the support structure and a protrusion on the inner cover. The divot may be a conical divot 304 and the protrusion may be a screw 306, where the conical divot is fixedly attached to the support structure and the screw is fixedly attached to the inner cover 312. The screw 306 may also be a type of pin instead of a conventional screw. Any object may be used in place of a screw as long the object fits into the conical divot 304 and functions in the manner described above. The pivot point may be adjustable such that the alignment of the inner cover can be changed if necessary. In one example, the pivot point may be adjusted by moving the position of the brackets.

As will be appreciated, the pivot point could be located in different locations to those illustrated in Figures 2 and 3. In an example, there may a single pivot point at the front end of the inner cover on top of the first end funnel 206A (rather than at the rear end).

The pivot point 204 may help to control movements of the inner cover 200 from the first position. Controlled movements of the inner cover 200 from the first position are advantageous such that the inner cover does not move into any other parts of the radiotherapy device. The pivot point 204 may also provide an alignment aid after servicing. During servicing, covers often have to be removed from the radiotherapy device so that service engineer may access the parts underneath. When the inner cover 200 is removed for servicing, it can be more easily replaced by aligning the support structure and the inner cover at the pivot point 204. The suspension system biases the protrusion upwards. Since the divot has a conical shape, if the protrusion abuts the divot at any point along the side of the cone, the suspension system and conical shape will guide the protrusion to the top point of the divot, and hence into position.

Since only small movements of the inner cover 312 are allowed, the pivoting motion at the pivot point 300 is also small. For example, the screw 306 and inner cover 312 may only move of the order of 1 degree from the central axis defined by the screw when the inner cover is in the first position.

Figure 4 depicts an inner cover 404 defining a bore, wherein the inner cover is movable from a first position. Figure 4 depicts one end of the inner cover 404 supported by two deformable leg supports 402A, 402B or "legs". The legs may be made of a flexible material. That is, a non-rigid material which allows for a bend or spring like effect to the legs. The flexible material of the legs should behave elastically, a force on the inner cover (and therefore the legs) may cause deformation to the legs, removal of the force allows the legs to return to their original position and inner cover to return to its first position. As will be appreciated, the flexible material of the legs could be metal. The material of the legs may be bended flat bar steel (for example, spring steel) or alternatively round bar steel could also be used. The material of the legs could also be a composite material, for example carbon or fiberglass composite.

The other end of the inner cover (not shown) may also be supported by two legs. The deformable leg supports may be fixedly attached to the underside of the inner cover 404. Figure 4 also depicts an additional flexible attachment 406 attached between the top side of the inner cover 404 and support structure. The attachment 406 may extend vertically upwards from the top side of the inner cover 404 to the support structure. The deformable leg supports may connect to the additional flexible attachment 406 via a circular support 408, the circular support extending around the circumference of the inner cover 404. The legs may rest on the floor or rest on top of the other components of radiotherapy device.

The inner cover 404 can be considered as suspended relative to the support structure by a suspension system. In the example of Figure 4, the suspension system comprises four deformable leg supports, two legs are shown 402A, 402B, and two legs are not shown. As would be appreciated, in other examples, the suspension system may comprise more or less deformable leg supports. For example, suspension system may comprise a single deformable leg support at the frond end of the inner cover such that only the front end of the inner cover is suspended. In general, the suspension system may comprise one or more deformable leg supports.

Figure 5 depicts an example radiotherapy device comprising a spring 502 and a module 506 according to the present disclosure. Figure 5 depicts the radiotherapy device support structure 516 and shows the radiotherapy device from a front-on view.

The spring 502 may be a vertical spring positioned in the radiotherapy device as shown in Figures 2a and 2b. The spring 502 may be fixedly attached to a first bracket 504, the first bracket 504 may be fixedly attached to a second bracket 512. The second bracket 512 may be fixedly attached to the support structure 516 of the radiotherapy device. The second bracket 512 may be fixedly attached to the support structure 516 via one or more screws 508A, 508B. The position of bracket may be adjusted by use of the screws 508A, 508B. The spring 502 may comprise a position adjuster 504. The position adjuster 504 can be adjusted to change the position of the spring.

As described in relation to Figures 2a and 2b, springs 502 may suspend the inner cover with respect to the radiotherapy device such that the inner cover 510 is movable from a first position. The springs may support the weight of the inner cover in the first position.

Vertical springs 502 may be arranged in the radiotherapy device as shown in Figures 2a and 2b. In the examples of Figures 2a and 2b, there are four springs 202A, 202B, 202C, 202D housed in brackets. The springs may suspend the inner cover with respect to the radiotherapy device. Each spring is vertically oriented with respect to the radiotherapy device. Two of the four springs 202A, 202B are positioned at one side of the inner cover and the other two springs 202C, 202D are positioned at the opposite side of the inner cover. Two of the four springs are positioned at the front end of the inner cover 202A, 202C and the other two springs are positioned at the rear end of the inner cover 202B, 202D. The front end of the inner cover is the end which receives the patient via the table 210. The springs 202A, 202B, 202C, 202D may attach to the inner cover and are housed in the support structure. The springs 202A, 202B, 202C, 202D may support the weight of the inner cover. Figure 4 illustrates an alternative suspension system.

According to the present disclosure, a variety of arrangements of a suspension system may allow the inner cover 200 to be movable from a first position. The arrangement used will depend on the radiotherapy device in question, for example the geometry of the ring-shaped gantry 104 or the table 110. In a first example, four vertical springs may be arranged as shown in Figures 2a and 2b. The skilled person will appreciate that other arrangements of springs could be used. For example, in a second example, there may be two vertical springs arranged such that one spring is positioned on one side of the inner cover and one spring is positioned on the other side of the inner cover. In a third example, there may be six vertical springs arranged such that three springs are positioned along one side of the inner cover and three springs positioned on the other side of the inner cover. In a forth example, there may be numerous horizontally orientated springs arranged on either side of the inner cover (for example, 20 springs or 40 springs). In a fifth example, there may be numerous springs attached to the inner cover where each spring is orientated radially outwards positioned around the circumference of the inner cover.

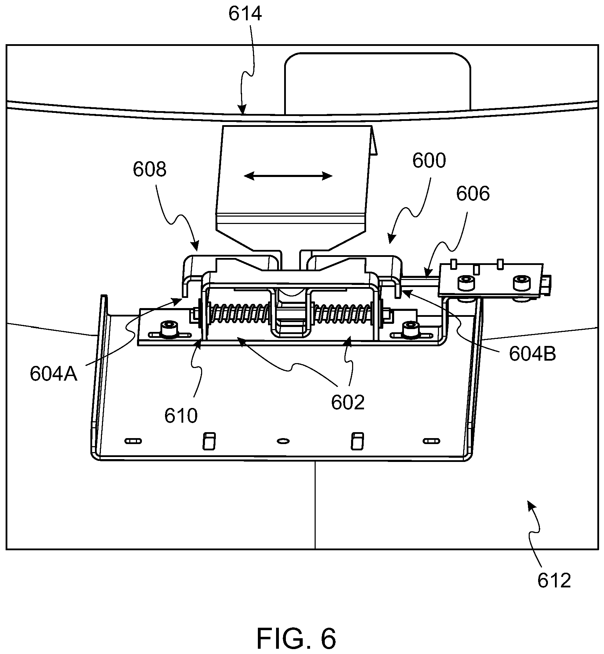

Figure 5 also depicts a module 506 according to the present disclosure. The module 506 may comprise one or more biasing mechanisms configured to return the inner cover to the first position. A close view of a module 506 depicting a module is provided in Figure 6.

Figure 6 depicts an example module 600 according to the present disclosure. One or more modules may be provided on a radiotherapy device having an inner cover which is movable from a first position. In the present disclosure, modules are attached between the inner cover and the support structure. The modules may comprise one of more of a number of features, which will each be described below in turn.

Firstly, a module 600 may comprise one or more biasing mechanisms 602 configured to return the inner cover to the first position. As explained in relation to Figures 2a and 2b, the inner cover is moveable from a first position according to the present disclosure. The inner cover may be moved from the first position by some force on the inner cover, for example, by the patient moving or an operator leaning into the inner cover. The nature of the inner cover suspended relative to the support structure by a suspension system is such that when the force on the inner cover is removed, the inner cover will be biased back in the vertical direction to return to the first position. For example, if the inner cover is suspended by vertical springs, the springs will return to their equilibrium position.

Alternatively or in addition to vertical movement, the inner cover may have been displaced from the first position in a horizontal direction. For horizontal movement of the inner cover, the vertical suspension springs would not effectively return the inner cover to the first position as the inner cover may move or swing for a period of time due to the momentum caused by the impact. The biasing mechanisms 602 are advantageous as they return of the inner cover to the first position in a reduced amount of time. Without the biasing mechanisms the inner cover may return to the first position only when all momentum in the vertical springs has been lost. Without the biasing mechanisms the inner cover may swing or move in small oscillations for long periods of time. These movements are unwanted as they might delay a treatment from starting. The biasing mechanisms 602 effectively have a damping effect to oscillations of inner cover, returning the inner cover to its equilibrium position (the first position).

Factors such as friction between end funnel seals and the inner cover with other covers of the radiotherapy device could hinder the precise return of the inner cover to the first position. The biasing mechanisms help to precisely return the inner cover to the first position after a movement of the inner cover. The biasing mechanisms 602 are advantageous as precise a return of the inner cover means that the inner cover diameter does not need to be decreased or the gantry size increased to provide greater margins between the cover and the rotating gantry parts to accommodate the inner cover design of the present disclosure. The biasing mechanism 602 may be a return spring. The return spring is a spring configured to return the inner cover precisely to its original position, to the first position.

There usually is only a small margin between the inner cover and rotating gantry components of the radiotherapy device. It is therefore very important that the inner cover returns to the first position before treatment starts. If the gantry parts start to rotate while the inner cover is not in the first position, there is a chance that the rotating parts may collide with the inner cover and become damaged. The biasing mechanisms 602 provide precise return of the inner cover to the first position and in a reduced amount of time.

It should be noted for radiotherapy devices with both return springs 602 and vertical support springs 502, the return springs and vertical springs have different functions are configured as such. Return springs 602 may overcome frictions within the radiotherapy device to precisely return the inner cover to the first position in a reduced time, whereas the support springs suspend the inner cover with respect to the support structure and support the weight of the cover. The return springs 602 are dimensioned as such and do not carry the weight of the inner cover. In the first position, return springs may be configured to be completely unloaded while the vertical springs 502 carry the weight of the inner cover. To achieve this, the position of the of the module 600 may be adjusted. When the inner cover is not in a first position, the return springs 602 may be loaded but do not carry the majority of the weight of the inner cover.

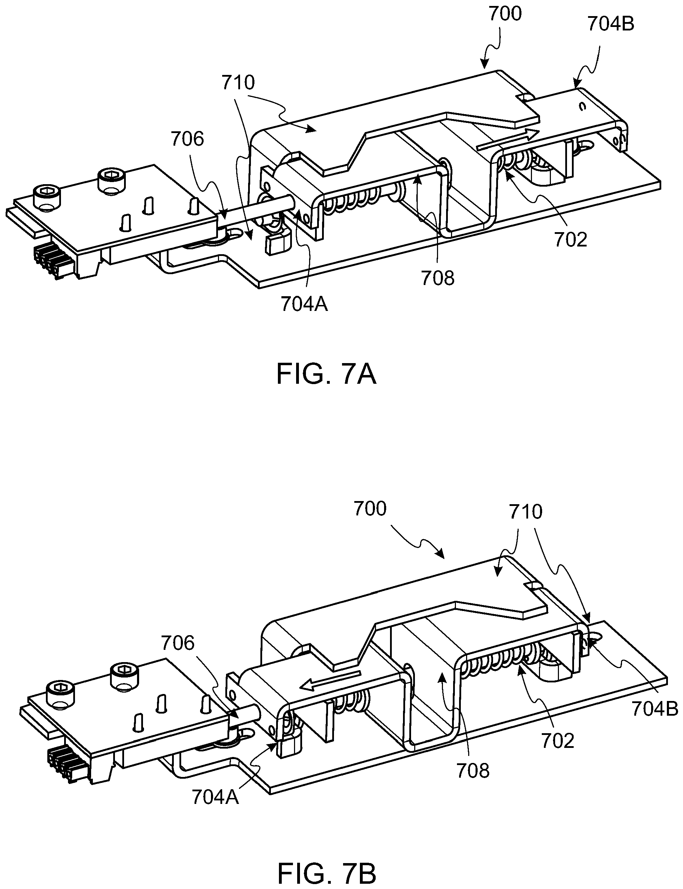

The module 600 may also comprise one or more end stops 604A, 604B, wherein the end stop is configured to prevent the inner cover from being displaced beyond a threshold distance. If the inner cover is displaced from the first position by too far a distance, the inner cover may risk of colliding with the gantry or other parts of the radiotherapy device. The end stops may therefore be positioned to prevent the inner cover colliding with the gantry. The end stops can be positioned on the biasing mechanisms to stop the biasing mechanisms from extending beyond a threshold distance. In other words, at a certain displacement of the inner cover, the inner cover will not be allowed to move any further. As will be appreciated, the end stops may be mechanical end stops which cannot move beyond a threshold distance. The end stops will be described in more detail with reference to Figures 7a and 7b.

The module 600 may also comprise a sensor 606 configured to provide displacement values indicative of the displacement of the inner cover from the first position. The sensor 606 may be configured such that the first position of the inner cover is the reference point for measuring changes in displacement. The sensor 606 may measure changes in position of the inner cover 614 from the support structure 612 of the radiotherapy device. As explained above, the support structure 612 of the radiotherapy device remains stationary as the inner cover 614 is able is to be displaced from its first position.

The sensor 606 may be configured to measure displacement in a linear fashion. The sensor 606 may be configured to measure displacement from the first position of the inner cover in either a horizontal or vertical direction with respect to the support structure 612 of the radiotherapy device. The sensor 606 may be potentiometer. A potentiometer has good radiation durability so will need to be replaced less often than other types of sensor.

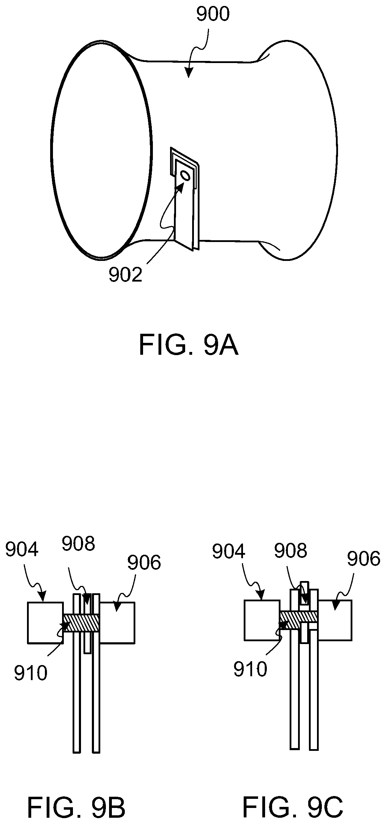

Alternative sensor configurations are also provided. For example, an inductive sensor, a capacitive sensor, photoelectric sensors, a switch sensor, a time of flight camera, contact sensor or a theremin sensor. More detail on example sensor configurations are given with reference to Figures 9a - 10b. In some examples, sensors may form part of the module 600. In other examples, sensors may not form part of the module. The preferable position and configuration of the sensor depends on the type of sensor.

It should be emphasised that modules may optionally comprise any combination of the features of end stops, biasing means and displacement sensors. A module according to the disclosure may include all three features, may include only one of the features, or may include any combination of two of the three mentioned features. For example, the module may include one or more end stops configured to prevent the inner cover from being displaced beyond a threshold distance and/or a sensor configured to provide displacement values indicative of the displacement of the inner cover. For example, some modules in a radiotherapy device may comprise end stops and no sensor whilst other modules in the same radiotherapy device comprise a sensor and no end stops. Any combination of components may be possible with the radiotherapy device.

The module 600 may be considered as comprising a first and a second part. A first part 608 of the module is fixedly attached to the inner cover 614 and a second part 610 of the module is fixedly attached to the support structure 612. The first 608 and second 610 parts of the module may be configured such that displacement of the inner cover 614 with respect to the support structure 612 causes displacement of the first part with respect to the second part. That is, the second part 610 of the module remains stationary with the fixed support structure 612 of the radiotherapy device while the first part moves with the inner cover, when the inner cover moves from the first position.

The first 608 and second 610 parts of the module may be configured such that the sensor 606 measures displacement of the inner cover 614 from the first position of the inner cover. In one example, the sensor 606 is housed on the second part of the module and a sensor probe is attached to the first part of the module such that the probe moves with the inner cover and is indicative of the inner cover's displacement from the first position.

The first and second parts of the module may also be configured such that the one or more biasing mechanisms 602 are configured to return the inner cover to the first position. In one example, the biasing mechanisms are housed on the second part 610 of the module and the biasing mechanisms themselves are part of the first part 608 of the module. For example, if the biasing mechanisms are return springs then the return springs can be considered as a component of first part 608 of the module. As the inner cover moves, the return springs will also move.

The first 608 and second 610 parts of the module may also be configured such that the end stops 604A, 604B are configured to prevent the inner cover from being displaced beyond a threshold distance. In one example, the end stop 604A, 604B is a component of the first part 608 of the module. The end stop 604A, 604B may be a stopper or a tab which catches or locks onto the second part 610 of the module. The stopper or tab may be configured such that it catches or locks onto the second part 610 of the module after a threshold distance is moved by the first part 608 of the module. At this point the first part 608 of the module cannot move anymore (and therefore the inner cover can also not move more than this threshold distance). Alternatively, the end stop may be a component of the first part of the module and may catch or lock onto the second part 610 of the module. Figure 7 depicts an example operation of two end stops.

Figures 7a and 7b depict example end positions of two end stops . Figure 7 depicts an example module 700 according to the present disclosure. The module 700 may comprise two return springs 702 configured to return the inner cover (not shown) precisely to the first position. The module 700 comprises one or more end stops 704A, 704B, wherein the end stops are configured to prevent the inner cover from being displaced beyond a threshold distance. The end stops 704A, 704B may be positioned on the return springs 702A, 702B, to stop the return springs (and therefore the inner cover) from extending beyond a threshold distance. The module 700 may also comprise a sensor 706 configured to provide displacement values indicative of the displacement of the inner cover from the first position. The sensor 706 is configured such that the first position of the inner cover is the reference point for measuring changes in displacement. The sensor 706 may measure changes in position of the inner cover from the support structure. As explained above, the support structure remains stationary as the inner cover is moved from its first position.

Similarly to the module 600 depicted in Figure 6, module 700 depicted in Figure 7 may be considered as comprising a first and a second part. A first part 708 of the module is fixedly attached to the inner cover and a second part 710 of the module is fixedly attached to the support structure. The first 708 and second 710 parts of the module may be configured such that displacement of the inner cover with respect to the support structure causes displacement of the first part with respect to the second part. That is, the second part 710 of the module remains stationary with the fixed support structure while the first part moves with the inner cover, when the inner cover is moved from a first position.

The first and second parts of the module may be configured such that the end stops prevent the inner cover from being displaced beyond a threshold distance. In this example, the two end stops 704A, 704B are components of the second part 710 of the module. The end stop may be a stopper or a tab as shown in Figure 7a and Figure 7b which catches onto the first part 708 of the module. The stopper or tab may be configured such that it catches or locks onto the second part 710 of the module after a threshold distance is moved by the first part 708 of the module. At this point the first part 708 of the module cannot move anymore (and therefore the inner cover can also not move more than this threshold distance).

In alternative examples, the maximum compression of the springs may act as a mechanical end stop. As will be appreciated, fully compressed springs can act in a similar manner to a stopper or tab configuration. In these examples, the spring itself acts as a mechanical end stop.

Figure 7a depicts the first part 708 of the module moving a threshold distance in a first direction and Figure 7b depicts the first part 708 of the module moving a threshold distance in the a second direction, where the first and second directions are opposite to each other. That is, Figures 7a and 7b show the two end positions of the first part 708 of the module, the furthest distances the first part 708 of the module is allowed to move. The maximal distances of the first part 708 of the module are representative of the maximal distances the inner cover is allowed to move. For some modules 700, the first direction may be horizontally right and the second direction may be horizontally left. For other modules 700, the first direction may be as vertically upwards and the second direction may be vertically downwards.

In Figure 7a the first part 708 of the module is at the threshold distance in the first direction. The end stop 704A at the opposite side of the module has caught the second part 710 of the module. The end stop 704A produces a locking effect and the first part of the module cannot move any further in the first direction. Similarly, Figure 7b the first part 708 of the module is at the threshold distance in the second direction. The end stop 704B at the opposite side of the module has caught the second part 710 of the module and it cannot move any further in the second direction.

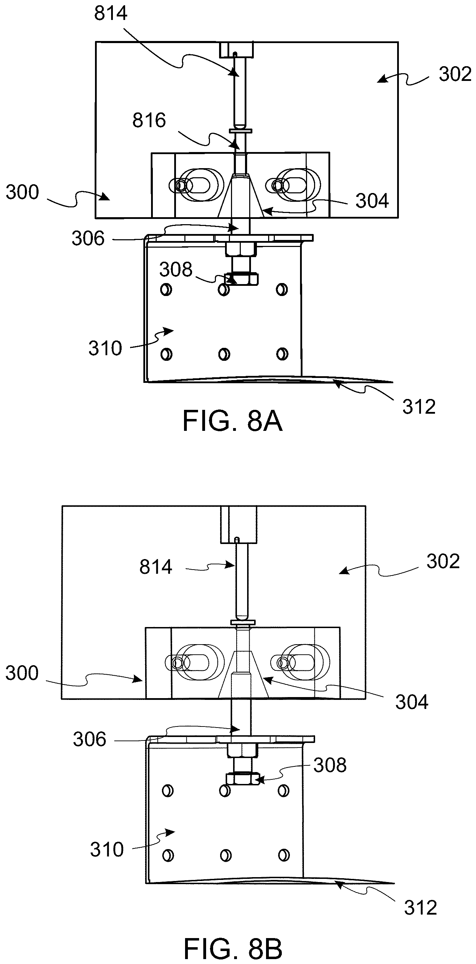

Figures 8a and 8b depict the pivot point of Figure 3 further comprising a sensor 814. The pivot point may also comprise a probe 816. The sensor 814 senses linear movement of the protrusion, for example the screw 306. As explained in relation to Figure 3, the screw 306 moves with the inner cover 312. Therefore the sensor 814 senses the linear movement of the inner cover, in a certain linear direction. If the inner cover moves downwards, the screw 306 may also move downwards, the sensor 814 measures the displacement moved by the inner cover in that linear direction. Displacement is measured with respect to the first position of the inner cover. The sensor 814 may rest directly on the screw 306 in order to detect changes in displacement. Alternatively, the sensor 814 may rest on a probe 816 the probe resting on the screw 306. The inner cover moves in the linear direction, moving the probe 816 and in turn moving the sensor 814 such that the displacement of the inner cover can be measured.

Figure 8a depicts the inner cover 312 in the first position and the screw in its engaged position. Figure 8b depicts the inner cover 312 after a displacement of the inner cover.

As explained above, a sensor may be used to detect displacement of the inner cover from the first position. Figures 6, 7a and 7b and 8 depict a linear potentiometer sensor. Figures 6, 7a and 7b depict a module comprising a linear potentiometer sensor.

Alternative sensors for detecting displacement of the inner cover from the first position are also provided herein. Sensors may form part of a module or may be separate from the modules, for example by attaching to another part of the inner cover.

Reference will now be made to Figures 9a, 9b and 9c which depict a photoelectric sensor configuration. One or more photoelectric sensors 902 may be configured to sense displacement of the bore when it moves from the first position. A photoelectric sensor 902 may comprise a light transmitter 904 and a light receiver 906. The light transmitter 904 and light receiver 906 may be fixed in place and will not move with the inner cover 900. For example, the light transmitter 904 and light receiver 906 may be fixed to the support structure (not shown). As part of a module or otherwise, the inner cover 900 may comprise an attachment comprising a hole 908, for example a flat, opaque piece of material comprising a hole.

While the inner cover 900 is in the first position, light 910 passes from the light transmitter 904 directly through the hole 908 to the light receiver 906. When the inner cover 900 is in the first position, the aperture of the light transmitter 904, the aperture of light receiver 906 and the centre point of the hole 908 are in line with each other. This is depicted in Figure 9b. When the inner cover 900 is in the first position, the light receiver 906 detects a maximum light intensity. The diameter of the hole 908 may be approximately equal to the beam diameter of the light transmitted. The photoelectric sensor system may be calibrated by measuring the light intensity when the inner cover is in the first position.

When the inner cover 900 moves away from the first position, the hole 908 is no longer in line with the light transmitter 904, less light 910 can pass through the hole and the intensity of the light detected by the receiver 906 is less. This is depicted in Figure 9c. In this manner, the photoelectric sensor can detect displacements of the inner cover 900 away from the first position in any direction within the plane perpendicular to the direction of the light.

As will be appreciated by the skilled person, fibre optics may be connected to the light transmitter and receiver. As will also be appreciated, the photoelectric sensor may be a light gate, detecting when the light is "cut off" or at a reduced intensity.

The attachment piece comprising a hole 902 may form part of one of the modules described herein. For example, the attachment piece comprising a hole 902 may be attached to the first part (the moving part) of the module. In other examples, the attachment piece is attached to another part of the inner cover 900.

In another example (not depicted), the sensor may be a time of flight camera which measures the displacement of the bore from the first position. The time of flight camera may be a 3D camera and use infrared light to reflect from the surface of the inner cover. Of course, other wavelengths of light may also be used. The time of flight camera can be positioned away from the inner cover, for example on the fixed support structure. Only one time of flight camera may be necessary to sense the displacement of bore as it moves away from the first position. In other examples, more than one time of flight cameras may be used. As will be appreciated, markers on the surface on the inner cover may be used in order to better detect the displacement of the bore from the first position.

In another example (not depicted), the sensor may be a laser distance gauge. As would be appreciated, the laser distance gauge acts in a similar way to the time of flight camera. The laser distance gauge can be positioned away from the inner cover, for example on the fixed support structure. The laser distance gauge may be a 2D gauge. Preferably, more than one laser distance gauge is used to detect displacement of the bore. The one than one laser distance gauges placed at different positions around the inner cover. For example, two laser distance gauges positioned detect displacement at the front of the inner cover and two laser distance gauges positioned to detect displacement at the rear of the inner cover.

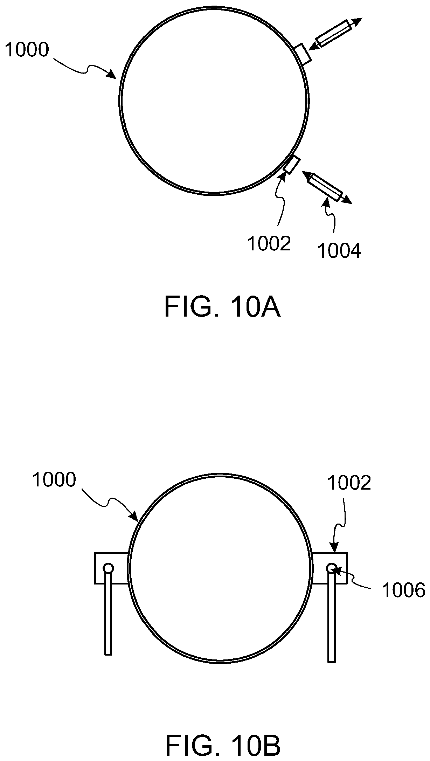

In other examples, sensors may be used to detect displacement of the inner cover from the first position based on electric or magnetic principles. For example, the sensor may be an inductive or capacitive sensor. Reference will now be made to Figure 10a which depicts a sensor 1004 which may be an inductive or capacitive sensor.

The inner cover 1000 may comprise one or more attachments comprising a measuring point 1002 made of a conductive material. The measuring point may be a plate, pin or simply a piece of conductive material. The conductive material may be a metal, for example. An inductive or capacitive sensor 1004 may be attached in a fixed position and will not move with the inner cover 1000, for example as part of the fixed support structure (not shown). The inductive or capacitive sensor 1004 measures distance to the measuring point of conductive material. The inductive or capacitive sensor measures distance in a linear direction.

In one example (not depicted), a theremin sensor may be used. In this example, the measuring point of conductive material is preferably a pin which extends from the inner cover. The theremin sensor detects changes in displacement of the measuring point and therefore the inner cover.

In another example, a contact sensor 1006 may be used. This example is depicted in Figure 10b. As will be appreciated, a contact sensor 1006 is a simple two dimensional sensor. As previously described, the inner cover 1000 is movable from the first position. The contact sensor 1006 is configured to detect movement in the plane depicted in Figure 10b. The contact sensor 1006 may comprise concentric rings, each ring with different electrical properties. The measuring point 1002 of the conductive material is preferably a pin, the pin moves when the inner cover is displaced and the pin moves around the rings, making a closed circuit with a particular ring depending on the displacement. Each ring represents a different position away from the central position and can be identified by the measured voltage. The central position of the contact sensor corresponds to the first position of the inner cover 1000.

In another example, a switch sensor may be used such as a simple mechanical micro switch or magnetic switch. The measuring point for a switch sensor is a switch engagement point. A switch sensor does not provide detailed displacement information of the inner cover from the first position but it does indicate when the inner cover has moved to a certain threshold displacement. In this way, it provides useful displacement information.

In any of the above sensor examples, the measuring point made of conductive material may form part of a module or may alternatively be attached to another part of the inner cover. The sensor is preferably not attached to the inner cover, instead attached to a fixed structure such as the support structure.

More generally and as will be appreciated, in some examples, the module may comprise at least part of the sensor. In other examples, the module may not comprise a sensor.

Some example sensors are configured to measure displacement from the first position of the inner cover in one dimension. For example, the linear potentiometer, the inductive sensor, the capacitive sensor and the switch sensor.

Some examples sensors are configured to measure displacement from the first position of the inner cover in more than one dimension. For example, the photoelectric sensor, the time of flight camera, the contact sensor and the theremin sensor. The time of flight camera may measure displacement in three dimensions. The photoelectric sensor, the contact sensor and the theremin sensor typically measure displacement in two dimensions.

As explained previously, the inner cover may be suspended relative to the support structure by a suspension system. For example, the suspension system may be one or more springs, or one or more deformable leg supports. For either example suspension system, any sensor system described may be used, namely: a linear potentiometer, an inductive sensor or a capacitive sensor, a switch sensor, a time of flight camera, contact sensor or a theremin sensor. Of course, any other suitable sensor system may also be used.

Reference will now be made to Figure 4 to explain how different example sensors may be configured with the one or more deformable leg supports suspension system.

In the example of a linear potentiometer sensor and the one or more deformable leg supports suspension system, the linear potentiometer may be positioned at the top flexible attachment 406. The linear potentiometer may work in a similar way already described.

The one or more deformable leg supports suspension system may additionally use a load cell system for detecting displacement of the inner cover. The deformable leg supports 402A, 402B may stand on load cells, when then the inner cover is pushed downwards towards the load cells or sideways, the load cells register the displacement.

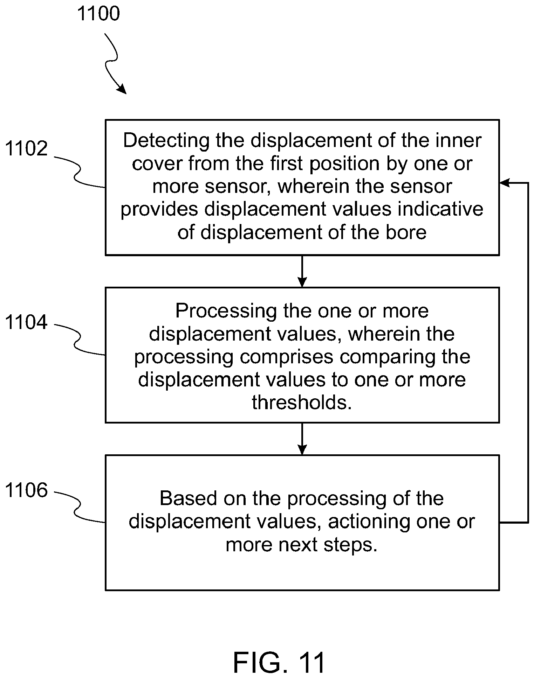

Reference will now be made to Figure 11. Figure 11 depicts a method 1100 for a radiotherapy device according to the present disclosure. The method begins from step 1102.

Step 1102 is detecting displacement of the inner cover from the first position by one or more sensors, wherein the sensor provides displacement values indicative of displacement of the inner cover. According to the present disclosure, the inner cover is movable from the first position. As explained above, the inner cover may be suspended relative to the support structure by a suspension system. For example, the suspension system may comprise one or more springs. For example, the suspension system may comprise one or more deformable leg supports. As would be appreciated by the skilled person, various suspension systems may be utilized to allow the inner cover to be moveable from the first position. The first position of the inner cover is the desired position of the inner cover whilst the patient is treated using the radiotherapy device.

Displacements of the inner cover may be caused when a person or piece of equipment comes into contact with the inner cover or otherwise causes a force to the inner cover. For example, the patient may shift position or sit up, pushing the inner cover and causing a displacement.. Movement of an operator, for example the operator reaching towards the patient to re-position the patient or to adjust the patient support, may push the inner cover and cause a displacement. A piece of equipment, for example a patient support, may touch the inner cover and cause a displacement.

According to the present disclosure, at least one module may comprise a sensor. The sensor may be configured such that the first position of the inner cover is the reference point for measuring changes in displacement. The sensor may measure changes in position of the inner cover from the support structure. Displacement values may be indicative of displacement of the inner cover in one certain direction. For example, displacement values may be measured from the first position of the inner cover in either a horizontal or vertical direction with respect to the support structure.

As will be appreciated, numerous displacement values may be detected, each displacement value detected by a sensor. Displacement of the inner cover may be defined by the detected displacement value/s. Sensors placed in different locations of the inner cover may provide displacement values indicative of displacement of the inner cover in that particular location and in a certain direction. For example, a sensor vertically aligned at the front of the inner cover provides displacement values indicative of displacement of the front of the inner cover and in a vertical direction.

In the example where the sensor comprises a photoelectric sensor arrangement, as will be appreciated, intensity values received by the light receiver may act as a displacement value.

Step 1104 is processing the one or more displacement values, wherein the processing comprises comparing the displacement values to one or more thresholds. Displacement values over a threshold may indicate that the inner cover has been displaced a substantial distance and that for such a movement, corrective action should be taken. For example, a patient may be entering into the bore on the patient table and touching the inner cover, pushing the inner cover and causing a displacement. Displacement values below a threshold value may indicate that inner cover is making small, inconsequential movements.

In the example where the sensor comprises a photoelectric sensor arrangement, the threshold may be an intensity threshold. The received intensity value dropping below the intensity threshold indicates that the inner cover has been displaced a substantial distance and that for such a movement, corrective action should be taken.

The thresholds are configured such that movements which are generally allowed and non-problematic can continue but movements which may potentially cause a problem if left to continue are flagged. Threshold distances may be of the order of millimetres, for example for movements in a vertical direction the threshold may be 2mm upwards or downwards and an for movements in a horizontal direction the threshold may be 2mm right or left. If there is more than one displacement value, then each displacement value may be compared to a different threshold value. The threshold/s will depend on the radiotherapy device in question, the typical components in and around the inner cover and the dimensions of the radiotherapy device. For example, a relevant dimension of the radiotherapy device is the bore diameter.

Step 1106 is based on the processing of the displacement values, actioning one or more next steps. As explained above, certain movements of the inner cover may be allowed (the displacement values below threshold/s) and other movements of the inner cover may have the potential for causing a problem (displacement values above threshold/s). Actioning a next step may involve a plurality of next steps. The next step/s may depend on conditions of the radiotherapy device (for example, whether a patient table is in motion or whether the gantry is in rotation) and/or on the location of the sensor detecting the displacement (for example, whether the sensor is at the front of the inner cover or at the rear of the inner cover).

For example, if the displacement value/s are more than a threshold value and the patient table is in motion, the next step may comprise stopping the motion of the patient table. If a patient is entering into the bore on the patient table and touching the inner cover then the motion of the patent table may stop to prevent further pushing of the inner cover.

For example, if the displacement value/s are more than a threshold value and the gantry is rotating, the next step may comprise stopping the gantry rotation. Stopping the gantry rotation may be beneficial so that the cause of the displacement can be removed before continuing gantry rotation and patient treatment. Stopping the gantry rotation may also prevent any damage to rotating gantry parts whilst the inner cover is displaced from the first position.

For example, if the displacement value/s are less than a threshold value, the next step may comprise maintaining the device as normal and sending a warning message to the user. In some examples, no warning message may be sent and the device is maintained as normal.

After step 1106, the method 1100 may repeat. The method 1100 may continuously operate. As will be appreciated, at a later point in time the inner cover may change position. For example, a force to the inner cover moving it from the first position may be removed, and the inner cover may return to the first position. At other points in time a new force to the inner cover may be applied. At step 1102, the new displacement of the inner cover may be detected by one or more sensors. At step 1104, the new displacement values may be compared to one or more thresholds. At step 1106, the one or more next steps may depend on conditions of the radiotherapy device.

For example, if the displacement value/s are less than a threshold value and if the motion of the patient table had been previously stopped due to the displacement of the inner cover, then the next step may comprise resuming the motion of the patient table. Similarly, the gantry rotation may also resume if the displacement value/s are less than a threshold value.

A radiotherapy device may comprise at least one module according to the present disclosure. The radiotherapy device may comprise a plurality of modules. There may be various configurations of at least one module according to the present disclosure. In different radiotherapy devices, there may be a different number of modules, the modules may positioned in different locations, the modules themselves may be configured differently and the modules may comprise different combinations of components. The number, position and configurations of the modules for the radiotherapy device in question may depend on factors such as the model of the radiotherapy device, the dimensions of the radiotherapy device and/or the quality level desired.

A first plurality of modules may be aligned in a first direction and a second plurality aligned in a second direction perpendicular to the first direction. A first plurality of modules may be aligned in a vertical direction and a second plurality aligned in a horizontal direction. Figure 5 depicts an example module 506 aligned in a vertical direction. Figure 6 depicts an example module 600 aligned in a horizontal direction. A module aligned in a first or second direction means that the module may sense the movement in that direction, the module may return the inner cover back to the first position in that direction, and the module may stop movement beyond a certain threshold in that direction.

Example configurations of the radiotherapy device will now be described. All examples are configurations are according to the present disclosure. For all example configurations, the inner cover is movable from a first position. That is, the inner cover is allowed to move in certain directions. The modules may or may not be connected to a system for allowing the inner cover to be movable from a first position. Various examples of such systems have already been given. As will be appreciated, the following example configurations of modules in a radiotherapy device do not present an exhaustive list.

In a first example configuration, the radiotherapy device comprises seven modules. This example is depicted in Figure 2a-2b. The seven modules are located such that there are four at the front end of the inner cover and three modules at the rear end of the inner cover. There may also be a single pivot point 204 located at the rear of the inner cover. In this example, four modules are aligned in a vertical direction 210A, 210B, 210C, 210D and three modules are aligned in a horizontal direction 210E, 210F, 210G. In this example configuration each module comprises two return springs, two end stops and a sensor. In this example configuration, the pivot point 204 does not contain a sensor. A configuration such as this may allow redundancy between certain displacement values detected by the sensor. That is, displacement values detected in the same direction may be able to be compared to another displacement values in the same direction for the purpose of reliability and accuracy checking. For example, if the inner cover moves vertically upwards, the two sensors at the rear of the inner cover aligned in the same vertical direction may have redundancy.

In a second example configuration, the radiotherapy device comprises seven modules. The seven modules are located such that there are four at the front end of the inner cover and three modules at the rear end of the inner cover. There may also be a single pivot point located at the topside of the rear of the inner cover. In this example, four modules are aligned in a vertical direction and three modules are aligned in a horizontal direction. In this example configuration five modules comprise a biasing mechanism, one or more end stops and a sensor and two modules comprise a biasing mechanism and one or more end stops. The two modules without a sensor are the two modules located at the rear of the inner cover and aligned in a vertical direction. In this example configuration, the single pivot point comprises a sensor. That is, for this configuration there are six sensors in total, five within five modules and one within a pivot point. A configuration such as this may allow to detect vertical displacement of the rear end of the inner cover using only the pivot point sensor, instead of two vertical sensors.

In a third example configuration, the radiotherapy device comprises five modules. The five modules are located such that there are four at the front end of the inner cover and one module at the rear end of the inner cover. There may also be a single pivot point located at the topside of the rear of the inner cover. In this example, two modules are aligned in a vertical direction, at the front of the inner cover, and three aligned in a horizontal direction, two at the front of the inner cover and one at the rear. In this example configuration, the single pivot point comprises a sensor. In this example configuration each module comprises a biasing mechanism, one or more end stops and a sensor.

Although the sensors can be part of the module, the sensor may alternatively be external to the module. The location of the sensor is dependent on the type of sensor used. For example, a time of flight camera may be external to the inner cover and not part of the module. Detailed description of the preferable positions of the sensors are described with reference to Figures 9a-10b.

It should also be noted that although the end stops are described as a part of a module, the end stops can also be designed elsewhere in the radiotherapy device. Other locations of the end stops will depend on the radiotherapy device in question.

The device and method of the present disclosure provide an improved bore design for a radiotherapy device. That is, the present disclosure provides an improved design for a radiotherapy device involving the inner cover and the bore of the radiotherapy device. It is possible for patients, operators or equipment to come into contact with the inner cover of the radiotherapy device. For a rigid inner cover, contact with the inner cover may cause pinching. People or objects may come between the inner cover and another piece of equipment such as the patient table. The radiotherapy device of the present disclosure is such that the inner cover is movable from a first position and comprises biasing mechanisms configured to return the inner cover to a the first position. This design allows the inner cover to move if anything is caught between the patient table and inner cover.

The biasing mechanisms help to precisely return the inner cover to the first position after a movement of the inner cover. Factors such as friction between end funnel seals and the inner cover with other covers of the radiotherapy device could hinder the precise return of the inner cover to the first position. There usually is only a small margin between the inner cover and rotating gantry components of the radiotherapy device. It is therefore very important that the inner cover returns to the first position before treatment starts. If the gantry parts start to rotate while the inner cover is not in the first position, there is a chance that the rotating parts may collide with the inner cover and become damaged. The biasing mechanisms provide precise return of the inner cover to the first position. The biasing mechanisms are advantageous as precise a return of the inner cover means that the inner cover diameter does not need to be decreased or the gantry size increased to provide greater margins between the cover and the rotating gantry parts to accommodate the inner cover design of the present disclosure.

In some examples, the module may also comprise one or more end stops. The end stops may therefore be positioned to prevent the inner cover colliding with the gantry. The end stops can be positioned on the biasing mechanisms to stop the biasing mechanisms from extending beyond a threshold distance. In other words, at a certain displacement of the inner cover, the inner cover will not be allowed to move any further. End stops may also be useful when mounting and adjusting the inner cover, for example as part of servicing. During servicing, the inner cover is likely to be moved and pushed and there is a possibility of the inner cover swinging and colliding into components of the gantry when the inner cover is mounted onto the radiotherapy device. The end stops prevent such collisions occurring.

The sensors of the present disclosure allow the movements of the inner cover from the first position to be monitored. The method 1100 of the present disclosure allows the movements to be monitored in a useful manner. By comparing the displacement values to thresholds, any pinching risk can be limited. For example, by stopping the patient table after a displacement, risk can be avoided.

The above implementations have been described by way of example only, and the described implementations and arrangements are to be considered in all respects only as illustrative and not restrictive. It will be appreciated that variations of the described implementations and arrangements may be made without departing from the scope of the invention.

A number of different sensors are provided, the sensors configured to provide displacement values indicative of the displacement of the inner cover from the first position. Different sensors present different advantages.

Linear potentiometers are durable against radiation and are relatively cheap.

Sensors such as laser distance gauges, time of flight cameras, inductive and capacitive sensors are advantageous as they are contact-less sensors. Contact-less sensors reduce the number of components necessary to use the sensor and generally will have a higher degree of precision, increased reliability and a longer lifetime. Laser distance gauges and time of flight cameras are additionally advantageous as they don't need additional attachments or measuring points to protrude from the inner cover.

The photoelectric sensor system described in relation to Figures 9a-9c is advantageous as the light transmitter and receiver could be placed away from the radiation source, they do not have to be in close proximity to the inner cover. This means the sensor may have reduced radiation exposure and therefore a longer lifetime.

Some sensors advantageously measure displacement of the inner cover in more than one dimension. For example, the photoelectric sensor, the time of flight camera, the contact sensor and the theremin sensor can measure displacement in two dimensions. Measuring displacement of the inner cover in more than one dimension with a single sensor may reduce the total number of sensors necessary in the radiotherapy device. The time of flight camera may measure the movements of the inner cover in three dimensions. As such, only one time of flight camera may be necessary to measure movements of the inner cover.

The following is a list of clauses of the disclosure.
1. A radiotherapy device comprising:
   a fixed support structure; and
   an inner cover defining a bore, wherein the inner cover is movable with respect to the first support structure from a first position.
2. The radiotherapy device of clause 1 further comprising a rotatable ring-shaped gantry.
3. The radiotherapy device of any preceding clause, wherein the inner cover is suspended relative to the support structure by a suspension system.
4. The radiotherapy device of clause 3, wherein the suspension system comprises one or more of: one or more springs, and one or more deformable leg supports.
5. The radiotherapy device of any preceding clause, wherein the inner cover is movable from the first position in two dimensions, in the plane of the circular cross section of the inner cover.
6. The radiotherapy device of any preceding clause further comprising at least one module comprising one or more biasing mechanisms configured to return the inner cover to the first position.
7. The radiotherapy device of clause 6, wherein the one or more biasing mechanisms are return springs.
8. The radiotherapy device of clause 6 or 7, wherein the at least one module further comprises one or more end stops, wherein the one or more end stops are configured to prevent the inner cover from being displaced beyond a threshold distance.
9. The radiotherapy device of clause 8, wherein the one or more end stops are positioned to prevent the inner cover colliding with components of the radiotherapy device.
10. The radiotherapy device of any preceding clause further comprising at least one sensor configured to provide displacement values indicative of the displacement of the inner cover from the first position.
11. The radiotherapy device of clause 10, wherein at least one sensor is configured to measure displacement from the first position of the inner cover in one dimension.
12. The radiotherapy device of clause 11 wherein the sensor is configured to measure displacement from the first position of the inner cover in either a horizontal or vertical direction with respect to the radiotherapy device support structure.
13. The radiotherapy device of clause 11 or 12, wherein the sensor is one of: a linear potentiometer, an inductive sensor, a capacitive sensor or a switch sensor.
14. The radiotherapy device of clause 10, wherein the sensor is configured to measure displacement from the first position of the inner cover in two dimensions.
15. The radiotherapy device of clause 14, wherein the sensor is a photoelectric sensor, a time of flight camera, a contact sensor or a theremin sensor.
16. The radiotherapy device of any of clauses 10 to 15, wherein the sensor is comprised in the module of clause 8.
17. The radiotherapy device of any preceding clause comprising a plurality of modules.
18. The radiotherapy device of any preceding clause comprising seven modules.
19. The radiotherapy device of any preceding clause further comprising a pivot point for pivotally restricting movement of the inner cover relation to the support structure.
20. The radiotherapy device of clause 19 comprising four modules at a front end of the inner cover, three modules at a rear end of the inner cover and one pivot point at the rear end of the inner cover.
21. The radiotherapy device of any preceding clause, comprising a first plurality of modules aligned in a first direction and a second plurality aligned in a second direction perpendicular to the first direction.
22. The radiotherapy device any preceding clause comprising at least one module, wherein a first part of the module is fixedly attached to the inner cover and a second part of the module is fixedly attached to the support structure,
   wherein the first and second parts of the module are configured such that displacement of the inner cover with respect to the support structure causes displacement of the first part with respect to the second part.
23. The radiotherapy device of clause 22 and any of clauses 10 to 16, wherein the sensor measures displacement of the first part relative to the second part such that the sensor measures displacement of the inner cover from the first position of the inner cover.
24. A module for the radiotherapy device of any of clauses 1 to 23, the module comprising a sensor configured to provide displacement values indicative of the displacement of the inner cover from the first position .
25. The module of clause 24 further comprising one or more biasing mechanisms configured to return the inner cover to the first position.
26. The module of clauses 24 or 25 further comprising one or more end stops, the one or more end stops configured to prevent the inner cover from being displaced beyond a threshold distance.
27. A method for a radiotherapy device, the radiotherapy device according to any preceding clause, the method comprising:
   detecting displacement of the inner cover from the first position by one or more sensors, wherein the sensor provides displacement values indicative of displacement of the inner cover;
   processing the one or more displacement values, wherein the processing comprises comparing the displacement values to one or more thresholds;
   based on the processing of the displacement values, actioning one or more next steps.
28. The method of clause 27, wherein if the displacement value is more than a threshold value and the patient table is in motion, the one or more next steps comprises stopping the motion of the patient table.
29. The method of clause 27 or 28, wherein if the displacement value is more than a threshold value and the gantry is rotating, the one or more next step comprises stopping the gantry rotation.
30. The method any of clauses 27 to 29, wherein if the displacement value is less than a threshold value, maintain the device as normal and send a warning message to the user.

## Claims

1. A radiotherapy device comprising:
a fixed support structure; and
an inner cover defining a bore, wherein the inner cover is movable with respect to the first support structure from a first position.

2. The radiotherapy device of claim 1 further comprising a rotatable ring-shaped gantry.

3. The radiotherapy device of any preceding claim, wherein the inner cover is suspended relative to the support structure by a suspension system.

4. The radiotherapy device of claim 3, wherein the suspension system comprises one or more of: one or more springs, and one or more deformable leg supports.

5. The radiotherapy device of any preceding claim further comprising at least one module comprising one or more biasing mechanisms configured to return the inner cover to the first position.

6. The radiotherapy device of claim 5, wherein the one or more biasing mechanisms are return springs.

7. The radiotherapy device of claim 5 or 6, wherein the at least one module further comprises one or more end stops, wherein the one or more end stops are configured to prevent the inner cover from being displaced beyond a threshold distance or to prevent the inner cover colliding with components of the radiotherapy device.

8. The radiotherapy device of any preceding claim further comprising at least one sensor configured to provide displacement values indicative of the displacement of the inner cover from the first position.

9. The radiotherapy device of claim 8, wherein the sensor is comprised in the module of claim 7.

10. The radiotherapy device of any preceding claim further comprising a pivot point for pivotally restricting movement of the inner cover relation to the support structure.

11. The radiotherapy device of claim 10 comprising four modules at a front end of the inner cover, three modules at a rear end of the inner cover and one pivot point at the rear end of the inner cover.

12. The radiotherapy device of any preceding claim, comprising a first plurality of modules aligned in a first direction and a second plurality aligned in a second direction perpendicular to the first direction.

13. The radiotherapy device any preceding claim comprising at least one module, wherein a first part of the module is fixedly attached to the inner cover and a second part of the module is fixedly attached to the support structure,
wherein the first and second parts of the module are configured such that displacement of the inner cover with respect to the support structure causes displacement of the first part with respect to the second part.

14. A method for a radiotherapy device, the radiotherapy device according to any preceding claim, the method comprising:
detecting displacement of the inner cover from the first position by one or more sensors, wherein the sensor provides displacement values indicative of displacement of the inner cover;
processing the one or more displacement values, wherein the processing comprises comparing the displacement values to one or more thresholds;
based on the processing of the displacement values, actioning one or more next steps.

15. The method of claim 14, wherein at least one of:
if the displacement value is more than a threshold value and the patient table is in motion, the one or more next steps comprises stopping the motion of the patient table; wherein if the displacement value is more than a threshold value and the gantry is rotating, the one or more next step comprises stopping the gantry rotation; and if the displacement value is less than a threshold value, maintain the device as normal and send a warning message to the user.
